# EUROPÄISCHE PATENTANMELDUNG

(11) **EP 1 510 219 A1**
(43) Veröffentlichungstag der Anmeldung: **02.03.2005**
(21) Anmeldenummer: 04024180.4
(22) Anmeldetag: 24.01.2001
(51) Int. Cl.: A61K 38/48, A61K 31/40, G01N 33/574, G01N 33/50, A61P 35/00, A61P 37/00

(54) **Verfahren zur Identifikation mindestens eines Aminopeptidasen-Inhibitors**

(30) Priorität: 24.01.2000 DE 10002820
(62) Teilanmeldung aus: 01911521.1
(71) Anmelder: MPB MelTec Patent- und Beteiligungsgesellschaft mbH, 39120 Magdeburg (DE)
(72) Erfinder: Schubert, Walter, 39175 Biederitz (DE)
(74) Vertreter: Hofstetter, Alfons J., Dr.rer.nat.

(57) **Zusammenfassung**

Die Erfindung betrifft ein Verfahren zur Identifikation mindestens eines Aminopeptidasen-Inhibitors und ein Verfahren zur Identifikation mindestens eines weiteren Inhibitors, der in Kombination mit dem mindestens einen Aminopeptidasen-Inhibitor wirkt.

## Beschreibung

Die vorliegende Erfindung betrifft ein Verfahren zur Identifikation mindestens eines Aminopeptidasen-Inhibitors und ein Verfahren zur Identifikation mindestens eines weiteren Inhibitors, der in Kombination mit dem mindestens einen Aminopeptidasen-Inhibitor wirkt.

Aminopeptidasen sind Enzyme der Zelloberfläche, die Peptide spalten. Sie werden von verschiedenen Zelltypen exprimiert. Ihre molekulare Funktion besteht u.a. in der Degradation biologisch aktiver Peptide. Die weiteren physiologischen, insbesondere die zellulären Funktionen der Aminopeptidasen sind bisher nicht geklärt. Jüngste Untersuchungen zeigen, daß Aminopeptidasen-Inhibitoren die Vermehrungsrate und die Invasion von Tumorzellen unterdrücken können. Diese Unterdrückung der Invasion wurde allgemein auf die proteolytische Aktivität von Zelloberflächen-assoziierten Aminopeptidasen zurückgeführt, die die extrazellulären Matrixproteine spalten, so daß die Tumorzellen in Organe eindringen und in diesen Organen wandern können. Zu den bekannten Aminopeptidasen-Inhibitoren gehören Actinonin, Bestatin und potente Inhibitoren vom Homophtalimid-Typ.

Bestatin kann gemäß J. Yoneda et al. Clin. Exp. Metastasis 10, 49-59, 1992 die Degradation des Typ IV Kollagens und damit die Invasion von Tumorzellen verhindern. Der genannten Publikation ist außerdem zu entnehmen, daß Bestatin keinen Einfluß auf die Tumorzelladhäsion und die Migration zu der extrazellulären Matrix besitzt.

Aus einer Veröffentlichung in Biol. Pharm. Bull. 22, 1010-1012, 1999 geht hervor, daß die Hemmung der Invasion durch einen Aminopeptidasen-Inhibitor vom Homophthalimid-Typ PIQ-22 auf eine Hemmung der Ausbildung von Zellextensionen zurückzuführen ist, wobei die Ursache dieser Hemmung unklar bleibt und eine Inhibition der Aminopeptidase N, im folgenden auch mit CD13, bezeichnet, durch PIQ-22 ausgeschlossen wird. Nach der genannten Veröffentlichung haben die beiden Aminopeptidasen-Inhibitoren Actinonin und Bestatin, die CD13 hemmen, gemäß *in vitro* Untersuchungen mit einem unspezifischen Matrigel-Analysesystem keine Wirkung auf die Tumorzellinvasion. Für Bestatin konnte auch keine Wirkung auf die Ausbildung von Zellextensionen nachgewiesen werden.

Besonders nachteilig ist die Tatsache, daß die bisher existierenden Untersuchungsmethoden nicht die Bedingungen *in vivo* widerspiegeln und daher häufig zu unbefriedigenden Ergebnissen führen, so daß die Wirkung der einzelnen Aminopeptidasen-Inhibitoren nicht umfassend aufgeklärt werden kann. Meist bleibt unklar, ob die Inhibition der Aminopeptidasen bei Tumoren *in vivo*, d.h. am Patienten, wirksam ist und ob nicht sogar bei bestimmten Tumorformen durch Inhibition der Aminopeptidasen ein gegenteiliger Effekt, bestehend in einer Potenzierung des Invasionsverhaltens in vivo, eintreten könnte.

Außerdem ist bei den Aminopeptidasen-Inhibitoren mit bekannter Wirkung der Wirkmechanismus völlig unbekannt, so daß keine neuen Substanzen entwickelt oder identifiziert werden können, die nur deshalb äußerst spezifisch wirken können, weil sie in bekannter Art und Weise in die Zeitfunktionen eingreifen. Beispielsweise konnte noch nicht geklärt werden, mit welchen anderen Proteinen in ein- und derselben Zelle Aminopeptidasen Wechselwirkungen eingehen und in welcher Weise diese Wechselwirkungen komplexe Zellfunktionen kodieren. Es ist daher auch nicht bekannt, ob und welche zellulären Mechanismen, die auf solchen Wechselwirkungen beruhen, ggf. durch eine Inhibition der Aminopeptidasen gezielt blockiert werden können und welche neuen Indikationen sich hieraus für einen klinischen Einsatz solcher Inhibitoren bzw. der aus diesen Inhibitoren weiter entwickelten Substanzen ergeben könnten.

Es ist daher Aufgabe der vorliegenden Erfindung ein Verfahren zur Identifikation mindestens eines solchen Aminopeptidasen-Inhibitors und ein Verfahren zur Identifikation mindestens eines weiteren Inhibitors, der in Kombination mit dem mindestens einen Aminopeptidasen-Inhibitor wirkt, bereitzustellen.

Eine der aufgeführten Aufgaben wird durch ein Verfahren zur Identifikation von Aminopeptidasen-Inhibitoren gelöst, die eine Blockierung einer Polarisierung von invasiven menschlichen oder tierischen Tumor- und/oder Immunzellen bewirken, wobei bei dem Verfahren zunächst Oberflächenproteinkombinationen eines Proteinnetzwerkes detektiert werden, die sich auf der Oberfläche der unbehandelten Tumor- und/oder Immunzellen befinden, wobei das Proteinnetzwerk bis zu 30 Oberflächenproteine aus einer Gruppe bestehend aus

| | | | | |
|---|---|---|---|---|
| 1. CD4 | 2. CD8 | 3. HLA-DR | 4. HLA-DQ | 5. CD3 |
| 6. CD26 | 7. CD38 | 8. CD45RA | 9. CD16 | 10. CD57 |
| 11. CD56 | 12. CD7 | 13. CD54 | 14. CD58 | 15. CD138 |
| 16. CD13 | 17. CD62L | 18. CD71 | 19. CD11 b | 20. CD36 |
| 21. CD29 | 22. CD49d | 23. CD18 | 24. CD49f | 25. CD19 |
| 26. CD2 | 27. CD20 | 28. CD10 | 29. CD44 | 30. CD80 |

umfaßt. In einem nächsten Schritt werden die oder gleichartige Tumor- und/oder Immunzellen mit mindestens einem Aminopeptidasen-Inhibitor behandelt. Anschließend werden die Oberflächenproteinkombinationen des Proteinnetzwerkes, die sich auf der Oberfläche der behandelten Tumor- und/oder Immunzellen befinden, detektiert und mit den Oberflächenproteinkombinationen des Proteinnetzwerks, die sich auf der Oberfläche der unbehandelten Tumor- und/oder Immunzellen befinden, verglichen. Der mindestens eine Aminopeptidasen-Inhibitor bewirkt bei einer Abweichung durch mindestens eine Veränderung des Oberflächenproteins CD13 die Blockierung der Polarisierung der Tumor- und/oder Immunzellen.

In einem zusätzlichen Schritt kann der mindestens eine identifizierte Aminopeptidasen-Inhibitor zu mindestens einer polarisierenden Tumor- und/oder Immunzelle zugegeben werden und die weitere Entwicklung der mindestens einen polarisierenden Tumor- und/oder Immunzelle detektiert werden, um so die tatsächliche Blockierung der Polarisierung nachzuweisen.

Das Verfahren kann weiterhin einen Kontrollschritt umfassen, bei dem die Bindung der unbehandelten Tumor- und/oder Immunzellen an organspezifische Endothelzellen und/oder an organspezifische extrazelluläre Strukturen detektiert wird, die Bindung der mit dem mindestens einen identifizierten Aminopeptidasen-Inhibitor behandelten Tumor- und/oder Immunzellen an die organspezifischen Endothelzellen und/oder die organspezifischen extrazellulären Strukturen detektiert wird und die detektierten Bindungen verglichen werden. Wird bei den behandelten Tumor- und/oder Immunzellen eine verminderte Bindung festgestellt, wird die Polarisierung besonders effektiv gehemmt, da eine wirkungsvolle organspezifische Adhäsion verhindert wird.

Eine der obigen Aufgaben wird durch ein Verfahren zur Identifikation von Inhibitoren gelöst, die in Kombination mit mindestens einem Aminopeptidasen-Inhibitor eine Blockierung einer Polarisierung von invasiven menschlichen oder tierischen Tumor- und/oder Immunzellen bewirken, wobei in dem Verfahren zunächst Oberflächenproteinkombinationen eines Proteinnetzwerkes, die sich auf der Oberfläche der unbehandelten Tumor- und/oder Immunzellen befinden, detektiert werden, wobei das Proteinnetzwerk bis zu 30 Oberflächenproteine aus einer Gruppe umfaßt, deren Zusammensetzung bereits oben aufgeführt ist. Die oder gleichartige Tumor- und/oder Immunzellen werden mit mindestens einem potentiellen Inhibitor behandelt und die Oberflächenproteinkombinationen des Proteinnetzwerkes, die sich auf der Oberfläche der behandelten Tumor- und/oder Immunzellen befinden, werden detektiert. Daraufhin werden die detektierten Oberflächenproteinkombinationen verglichen, wobei der mindestens eine Inhibitor bei einer Abweichung durch mindestens eine Veränderung eines Oberflächenproteins zur Blockierung der Polarisierung der Tumor- und/oder Immunzellen geeignet ist.

Die oder die gleichartigen Tumor- und/oder Immunzellen können neben dem mindestens einen Inhibitor auch mit mindestens einem Aminopeptidasen-Inhibitor behandelt werden, wobei die Kombination von dem mindestens einen Inhibitor und dem mindestens einen Aminopeptidasen-Inhibitor bei einer Abweichung der in den zwei verschiedenen Schritten detektierten Oberflächenproteinkombinationen durch mindestens eine Veränderung eines Oberflächenproteins CD13 die Blockierung der Polarisierung der Tumor- und/oder Immunzellen bewirkt.

Das Verfahren kann außerdem einen weiteren Schritt umfassen, bei dem der mindestens eine identifizierte Inhibitor oder eine Kombination aus dem mindestens einen identifizierten Inhibitor und dem mindestens einem Aminopeptidasen-Inhibitor zu mindestens einer polarisierenden Tumor- und/oder Immunzelle zugegeben wird und die weitere Entwicklung der mindestens einen polarisierenden Tumor- und/oder Immunzelle detektiert wird.

Vorteilhafterweise kann das Verfahren einen Kontrollschritt umfassen, in dem die Bindung der unbehandelten Tumor- und/oder Immunzellen an organspezifische Endothelzellen und/oder an organspezifische extrazelluläre Strukturen detektiert wird, in dem die Bindung der mit dem mindestens einen identifizierten Inhibitor und/oder mit einer Kombination aus dem mindestens einen identifizierten Inhibitor und dem mindestens einen Aminopeptidasen-Inhibitor behandelten Tumorund/oder Immunzellen an die organspezifischen Endothelzellen und/oder die organspezifischen extrazellulären Strukturen detektiert wird und in dem die detektierten Bindungen verglichen werden.

Grundsätzlich kann das Detektieren der Oberflächenproteinkombinationen Verfahrensschritte eines automatisierten Verfahrens zur Bestimmung von Molekülklassen, Molekülgruppen oder Molekülteilen in einem festen oder flüssigen Objekt gemäß der DE 197 09 348 C umfassen. Bei diesen Verfahrensschritten können in ein- und demselben Objekt, nämlich beispielsweise in einer Probe von Immunzellen und/oder Tumorzellen, Oberflächenproteine mittels sequentieller Aufbringung von Reagenzlösungen Yn (n = 1,2,3...N) mittels einer automatischen Vorrichtung untersucht und gemessen werden, wobei die Verfahrensschritte umfassen:
I. Aufnahme einer ersten Reagenzlösung Y1 aus einem Behälter, der die Reagenzlösung enthält,
II. Aufbringen der Reagenzlösung Y1 auf das Objekt, das sich auf einer objekttragenden Vorrichtung befindet,
III. Einwirken der Reagenzlösung über einen automatisch eingestellten Zeitraum,
IV. Aufnahme von mindestens einem Einzelmarkierungsmuster des zuvor mit der ersten Reagenzlösung Y1 markierten Objekts,
V. Wiederholung der Schritte I-IV durch Aufbringen der ersten Reagenzlösung Y1 oder einer zweiten Reagenzlösung Y2 oder eines Gemisches aus erster und zweiter Reagenzlösung, und
VI. Wiederholung der Schritte I bis V mit weiteren Reagenzlösungen Yn (n = 2,3...N) oder einem Gemisch davon und wobei
die in jedem Verfahrenszyklus erhaltenen Markierungsverteilungsmuster durch computergestützte Bildüberlagerung in ein komplexes molekulares Kombinationsmuster des zu untersuchenden Objekts überführt werden.

Aus diesem Kombinationsmuster können Informationen über das Vorhandensein der oben genannten Proteine gewonnen werden und damit auch die Oberflächenproteinkombinationen detektiert werden, wenn die verwendeten Reagenzlösungen markierte Substanzen enthalten, die gegen die entsprechenden Proteine gerichtet sind.

Bei den aufgeführten Kontrollschritten wird überprüft, ob die Polarisierung durch den mindestens einen Aminopeptidasen-Inhibitor und/oder den mindestens einen weiteren Inhibitor verhindert wird, indem die Bindung bestimmter Moleküle an definierte Strukturen gehemmt wird. Diese Kontrollschritte können durchgeführt werden, indem Immunzellen (Lymphozyten) und/oder Tumorzellen in Form eines kontinuierlichen Zellflusses in einem speziellen Gerät, das in der DE 199 32 158 A beschrieben wird, über mindestens eine Probe mit den definierten Strukturen geleitet werden. Während ohne Behandlung mit dem mindestens einen Aminopeptidasen-Inhibitor und/oder dem mindestens einen weiteren Inhibitor eine Bindung der Zellen an die definierten Strukturen erfolgen sollte, findet nach der Behandlung der Zellen mit dem mindestens einen Aminopeptidasen-Inhibitor und/oder dem mindestens einen weiteren Inhibitor keine Bindung oder eine verminderte Bindung an die Strukturen statt. Die Probe kann beispielsweise aus einem Organgewebeschnitt bestehen.

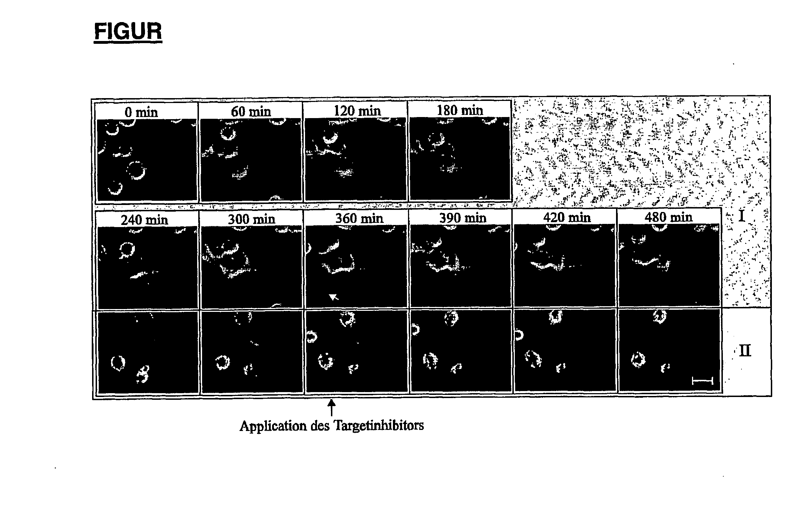

Weitere Merkmale und Vorteile der Erfindung gehen aus den im folgenden aufgeführten Untersuchungsergebnissen hervor, die unter anderem anhand von einer Figur beschrieben werden.
- Die Figur zeigt: in zeitlicher Abfolge Fotografien von polarisierenden Zellen, unbehandelt und mit einem Targetinhibitor behandelt.

Mit Hilfe zahlreicher Untersuchungen konnten die bereits oben aufgeführten 30 Zelloberflächenproteine identifiziert werden, die zu einem spezifischen Proteinnetzwerk gehören, das die frühen Phasen der Polarisierung von Tumorzellen und Immunzellen kontrolliert. In einer weiteren Untersuchung wurden nun Oberflächenproteinkombinationen dieses Proteinnetzwerkes von Karpas-Zellen unbehandelt und nach der Behandlung mit dem Aminopeptidasen-Inhibitor Actinonin detektiert. Zu diesem Zweck wurden zwei Gruppen V1 und V2 von Karpas-Zellen gebildet, wobei die Zellen der Gruppe V1 nicht behandelt wurden, während die Zellen der Gruppe V2 mit Actinonin behandelt wurden. In Tabelle 2 sind diejenigen Oberflächenproteinkombinationen zusammengestellt, die sowohl bei den mit Actinonin behandelten als auch bei den unbehandelten Zellen vorkommen. In dieser und den weiteren Tabellen 3 und 4 sind jedoch beispielhaft nur 18 der 30 Proteine aufgeführt, wobei die detektierten Proteine mit 1 gekennzeichnet sind und die nicht-detektierten Proteine mit 0 gekennzeichnet sind.

Die Proteine sind von 1 bis 18 durchnumeriert, wobei die Nomenklatur aus der Tabelle 1 hervorgeht.

**Tabelle 1**

| | | | | | |
|---|---|---|---|---|---|
| 1. CD2 | 2. CD3 | 3. CD4 | 4. CD8 | 5. CD16 | 6. CD56 |
| 7. CD57 | 8. CD26 | 9. CD38 | 10. CD71 | 11. HLA-DR | 12. HLA-DQ |
| 13. CD11 b | 14. CD45RA | 15. CD7 | 16. CD62L | 17. CD36 | 18. CD19 |

Die in den Tabellen 2 bis 4 angegebenen Zellanzahlen beziehen sich auf jeweils 1000 untersuchte Zellen in den Gruppen V1 und V2. In der Tabelle 2 ist eine Gesamtanzahl von 203 unterschiedlichen Proteinkombinationen aufgeführt.

In der Tabelle 3 sind die Oberflächenproteinkombinationen aufgeführt, die nur bei den unbehandelten Karpas-Zellen vorkommen und nie bei den Actinonin-behandelten Karpas-Zellen zu finden sind. Die Anzahl der in dieser Tabelle 3 bezeichneten Proteinkombinationen beträgt 131.

In der Tabelle 4 sind schließlich diejenigen Oberflächenproteinkombinationen aufgeführt, die ausschließlich bei den Actinonin-behandelten Karpas-Zellen vorkommen. Die Tabelle 4 enthält 60 verschiedene Proteinkombinationen.

Aus den Tabellen 2 bis 4 geht hervor, daß bei Betrachtung von lediglich 18 Proteinen der 30 Proteine insgesamt 394 unterschiedliche Oberflächenproteinkombinationen auftreten, wobei bei den unbehandelten Zellen insgesamt 334 unterschiedliche Kombinationen zu finden sind und bei den behandelten Zellen insgesamt 263 unterschiedliche Kombinationen detektiert werden können. Die hiermit nachgewiesene Änderung der Oberflächenproteinkombinationen führt zu einer spezifischen Blockierung der Zellpolarisierung.

Eine weitere Untersuchung wird anhand der Figur erläutert. In (I) ist der normale zelluläre Prozeß der Polarisierung einer Tumorzelle gezeigt. Durch *in vitro life i-maging* wird erfaßt, wie sich eine Sarkom-Zelle aus einer primär sphärischen Zellform heraus über die Bildung von 3 Zellextensionen (tripolare Zellform) und anschließende gezielte Rückbildung nur einer der drei Extensionen (weißer Pfeil bei 360 min) polarisiert. Die Definition einer Längsachse ist Voraussetzung für die darauf folgende Zellmigration. In (II) ist nachgewiesen, daß die Applikation eines selektiven Targetinhibitiors, hier ein monoklonaler Antikörper gegen eine extrazelluläre Domäne des CD13 (schwarzer Pfeil), die Zellpolarisierung vollständig verhindert. Die Zelle wird sphärisch und hoch adhäsiv, was aus dem Vergleich zwischen einer Fotografie der inhibierten Zelle (II) nach 480 min und einer Fotografie der nicht-inhibierten Zelle (I) nach 480 min (I) hervorgeht.

Ähnliche Ergebnisse erhält man bei dem Einsatz von Actinonin oder Bestatin als Targetinhibitor.

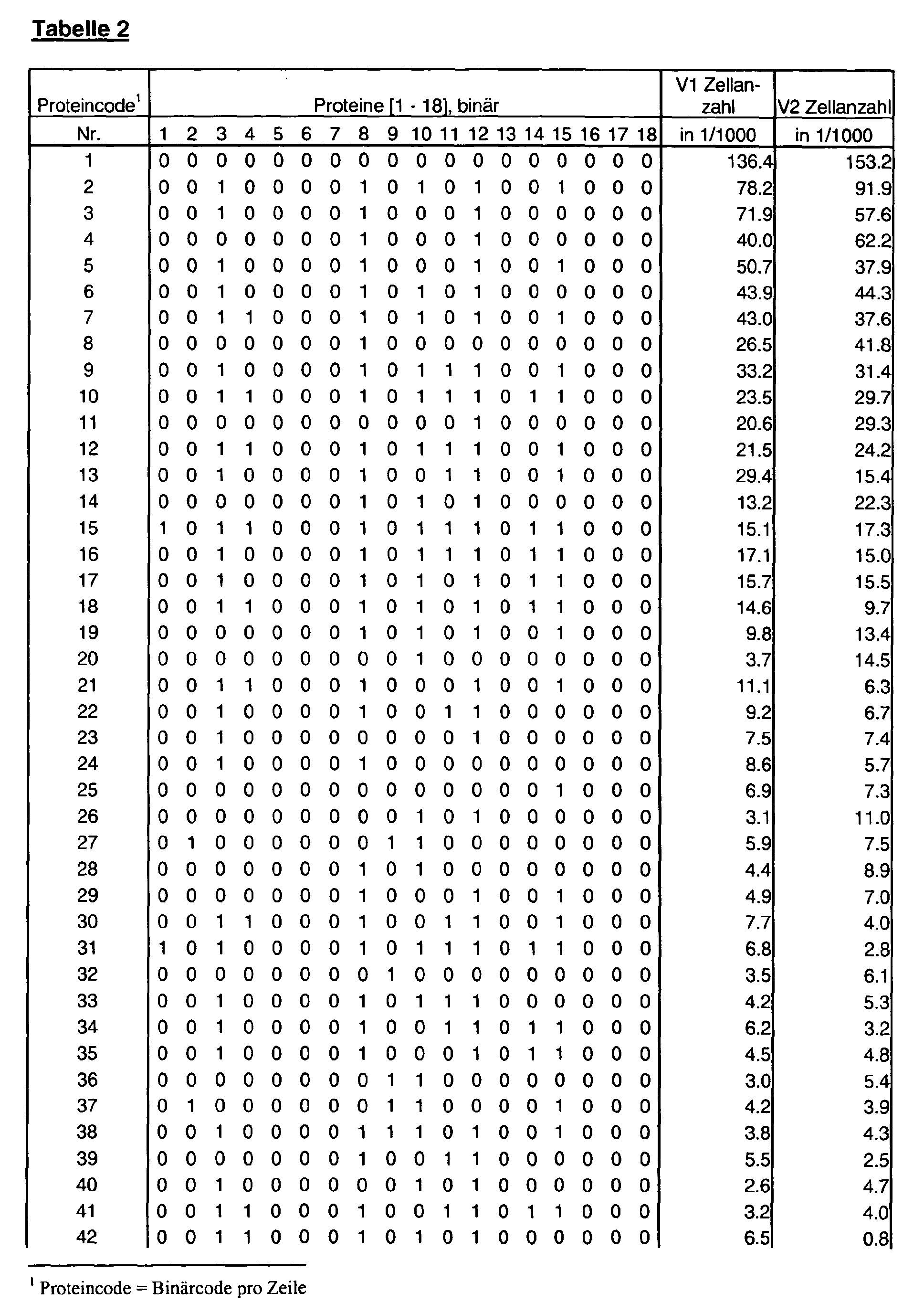

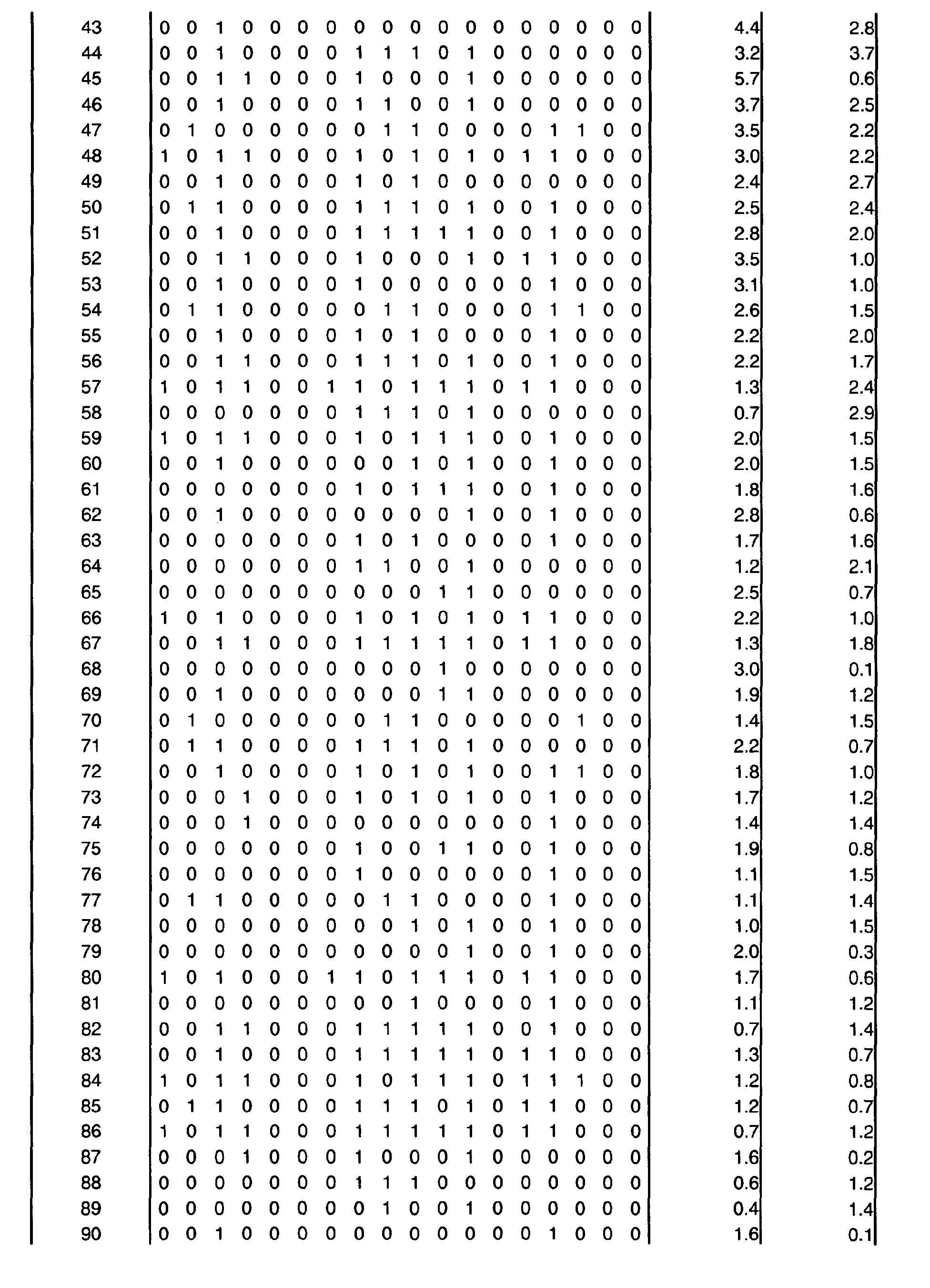

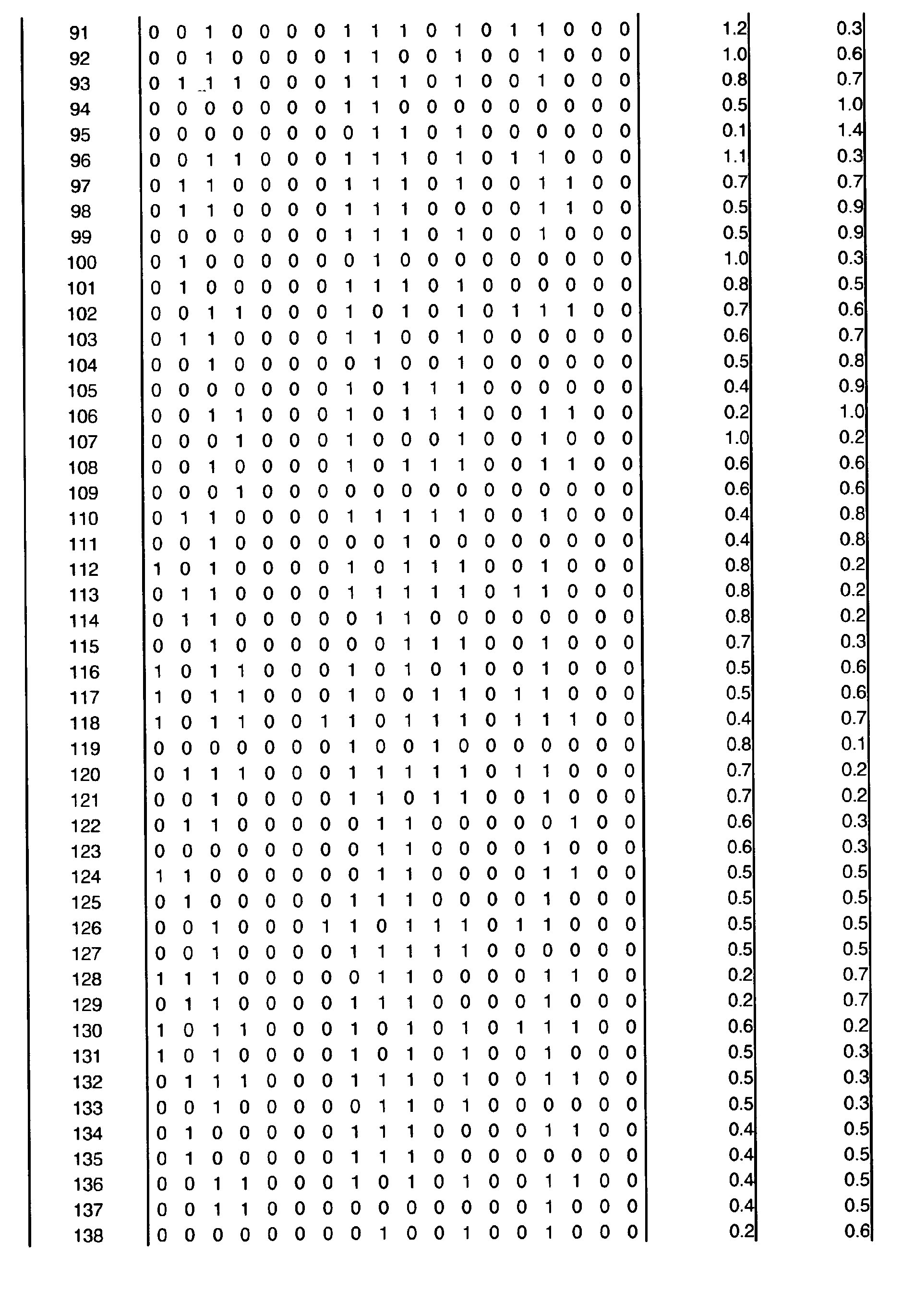

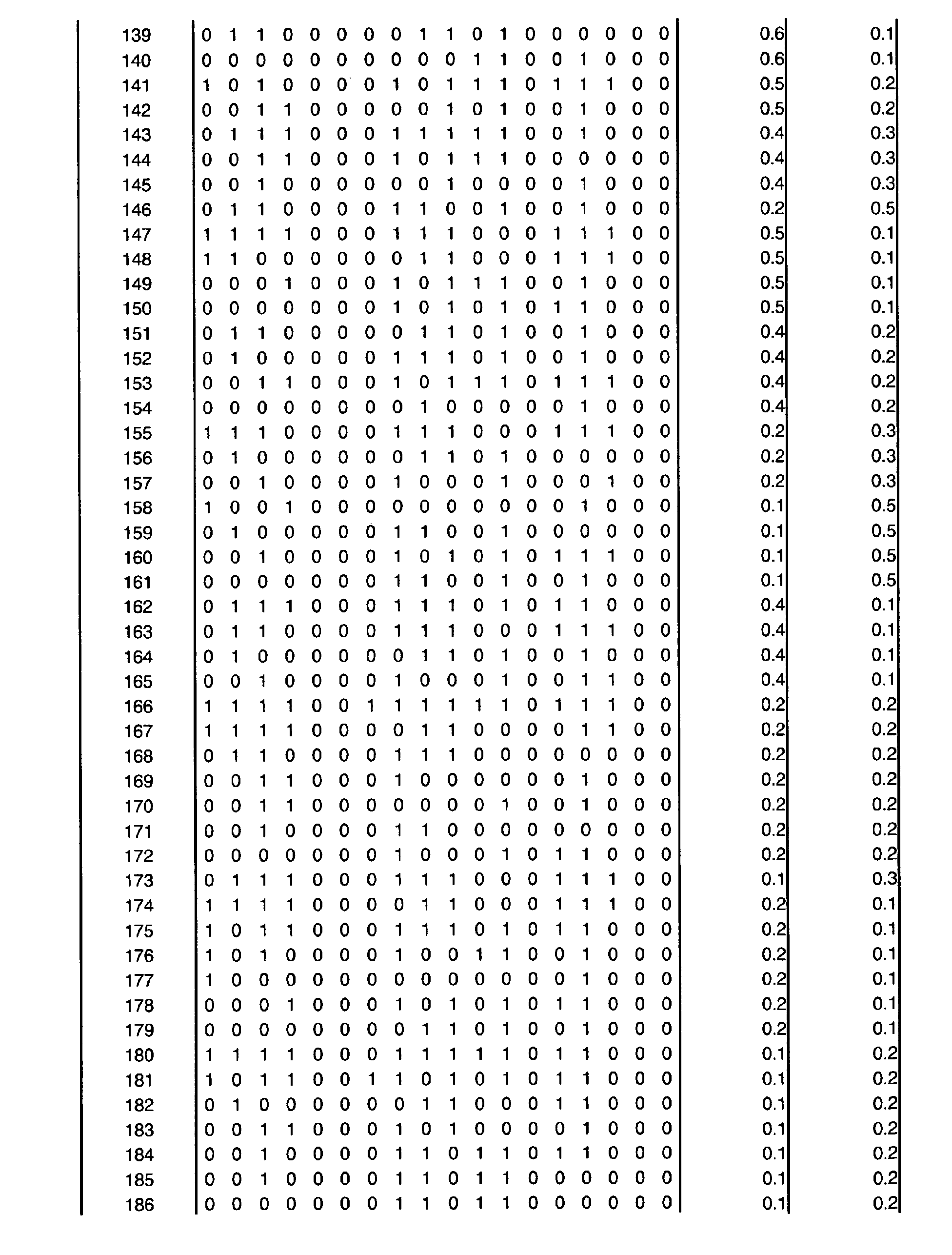

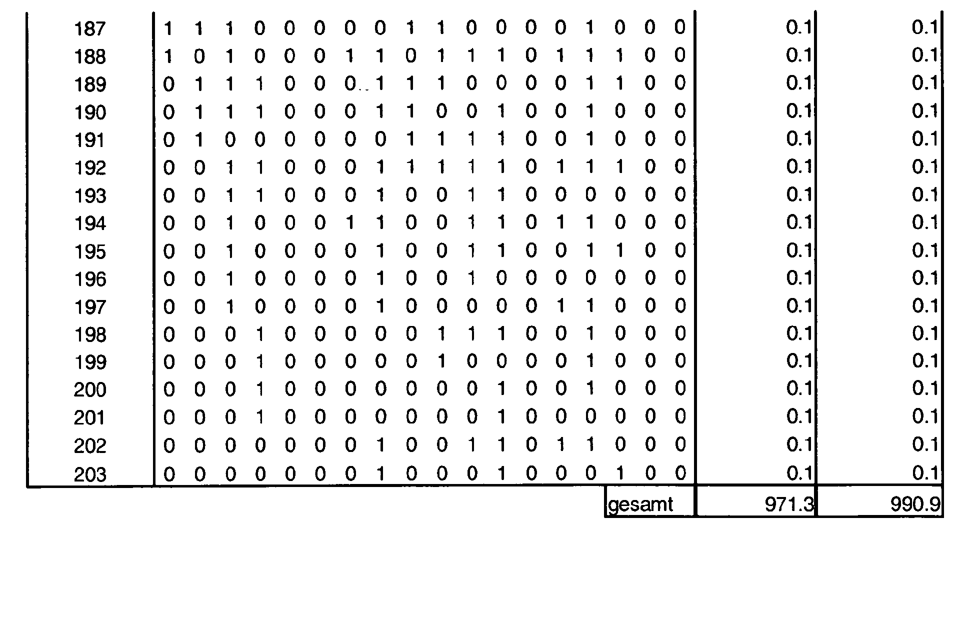

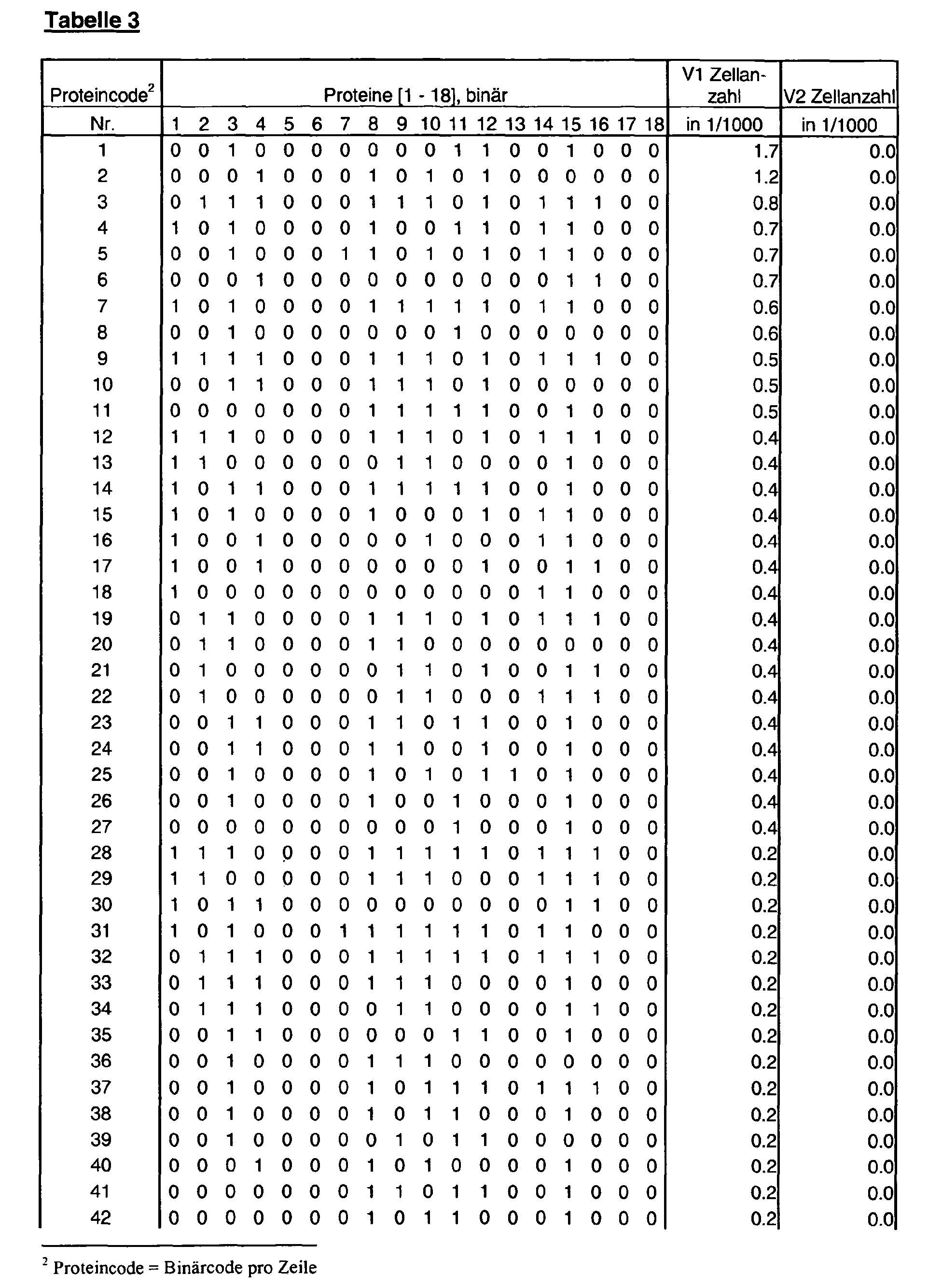

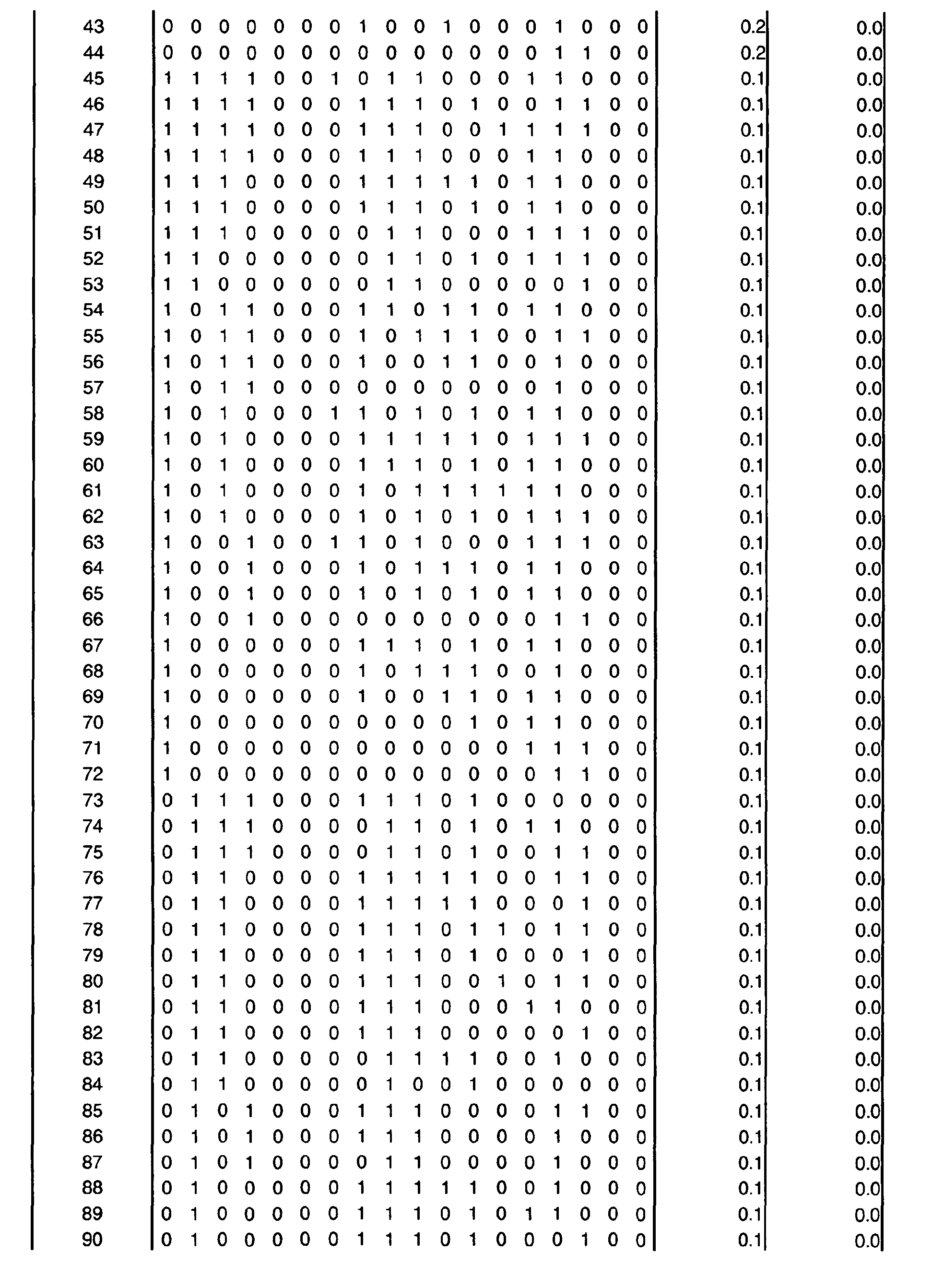

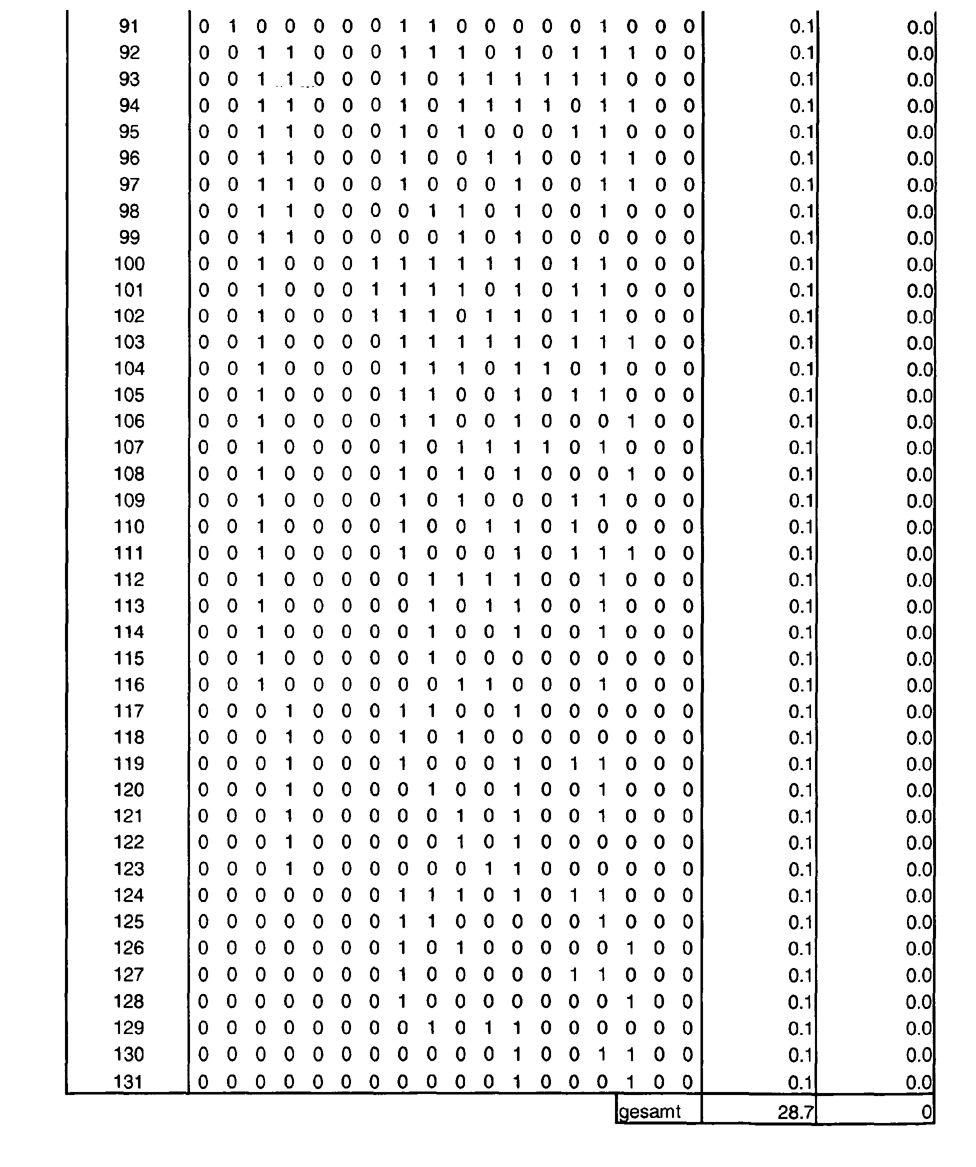

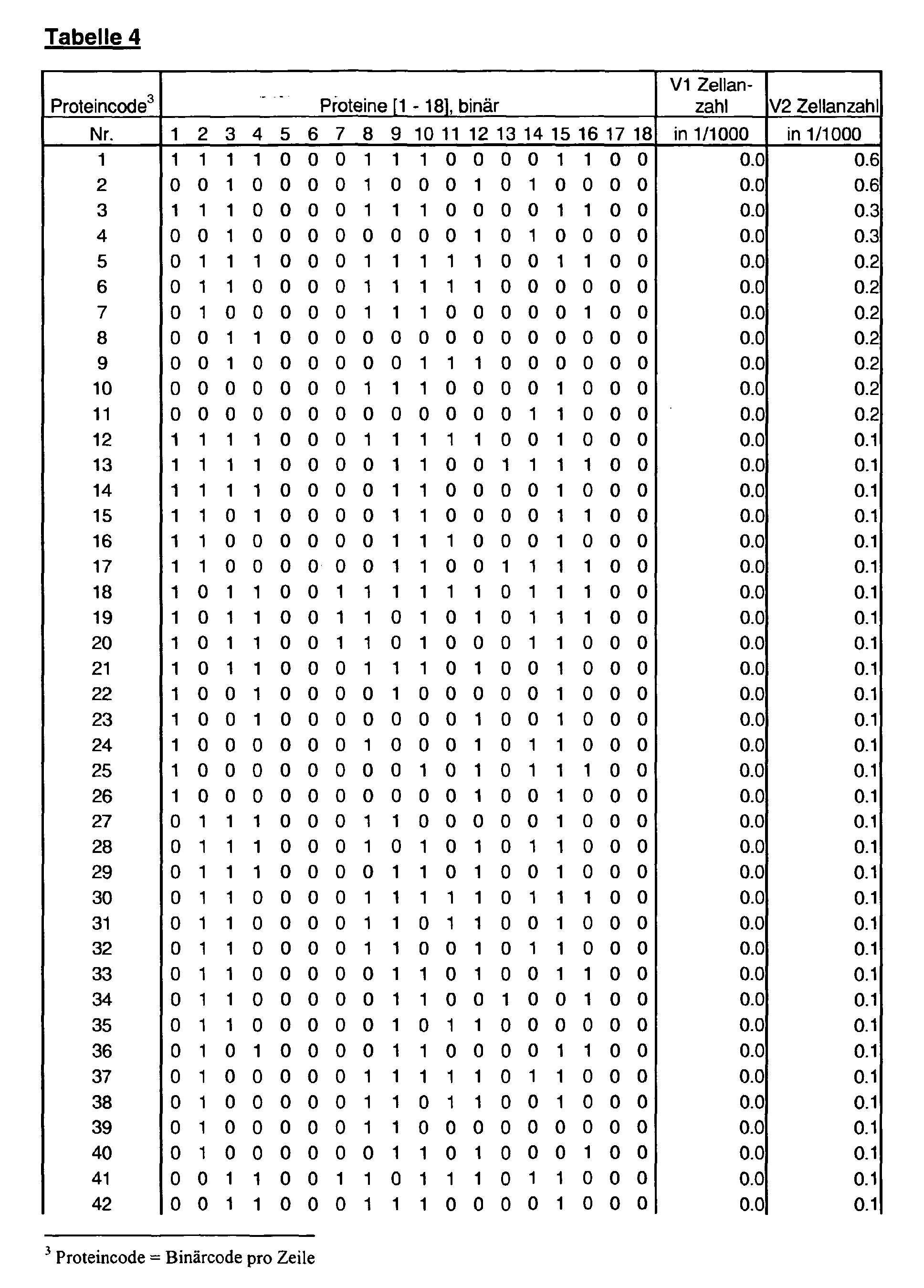

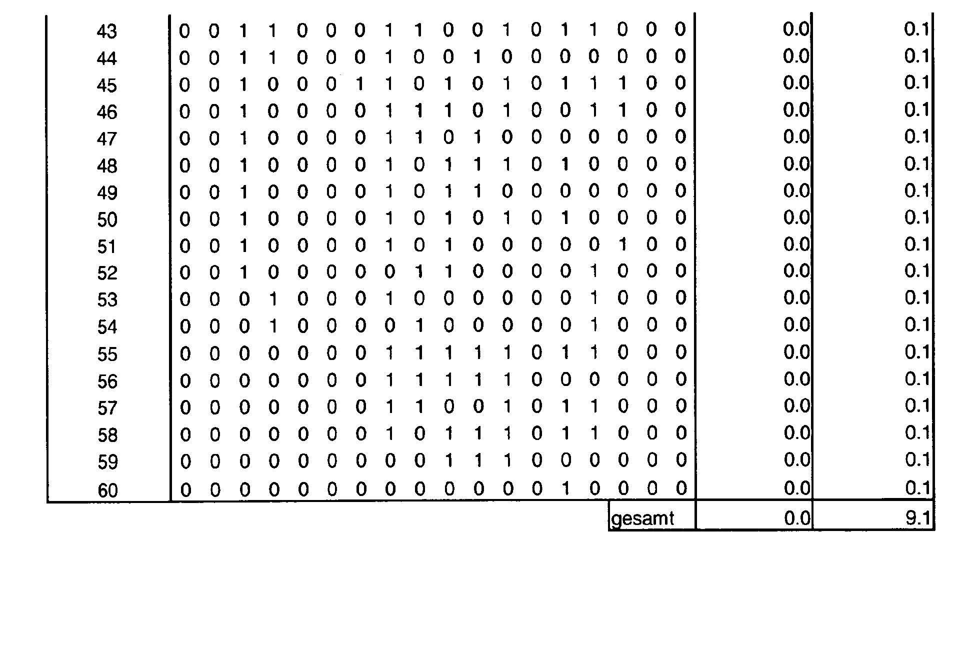

## Patentansprüche

1. Verfahren zur Identifikation mindestens eines Aminopeptidasen-Inhibitors, der eine Blockierung einer Polarisierung von invasiven menschlichen oder tierischen Tumor- und/oder Immunzellen bewirkt, folgende Schritte umfassend:
a) Detektieren von Oberflächenproteinkombinationen eines Proteinnetzwerkes, die sich auf der Oberfläche der unbehandelten Tumorund/oder Immunzellen befinden, wobei das Proteinnetzwerk bis zu 30 Oberflächenproteine aus einer Gruppe bestehend aus
| | | | | |
|---|---|---|---|---|
| 1. CD4 | 2. CD8 | 3. HLA-DR | 4. HLA-DQ | 5. CD3 |
| 6. CD26 | 7. CD38 | 8.CD45RA | 9. CD16 | 10. CD57 |
| 11. CD56 | 12. CD7 | 13. CD54 | 14. CD58 | 15. CD138 |
| 16. CD13 | 17. CD62L | 18. CD71 | 19. CD11 b | 20. CD36 |
| 21. CD29 | 22. CD49d | 23. CD18 | 24. CD49f | 25. CD19 |
| 26. CD2 | 27. CD20 | 28. CD10 | 29. CD44 | 30. CD80 |
umfaßt;
b) Behandeln der oder gleichartiger Tumor- und/oder Immunzellen mit mindestens einem Aminopeptidasen-Inhibitor;
c) Detektieren der Oberflächenproteinkombinationen des Proteinnetzwerkes, die sich auf der Oberfläche der behandelten Tumor- und/oder Immunzellen befinden; und
d) Vergleichen der in Schritt a) und in Schritt c) detektierten Oberflächenproteinkombinationen, wobei der mindestens eine Aminopeptidasen-Inhibitor bei einer Abweichung der in Schritt a) detektierten Oberflächenproteinkombinationen von den in Schritt c) detektierten Oberflächenproteinkombinationen durch mindestens eine Veränderung des Oberflächenproteins CD13 die Blockierung der Polarisierung der Tumor- und/oder Immunzellen bewirkt.

2. Verfahren nach Anspruch 1,
**dadurch gekennzeichnet,**
**daß** das Verfahren nach Schritt d) einen weiteren Schritt umfaßt, in dem der mindestens eine in Schritt d) identifizierte Aminopeptidasen-Inhibitor zu mindestens einer polarisierenden Tumor- und/oder Immunzelle zugegeben wird und die weitere Entwicklung der mindestens einen polarisierenden Tumor- und/oder Immunzelle detektiert wird.

3. Verfahren nach einem der Ansprüche 1 oder 2,
**dadurch gekennzeichnet,**
**daß** das Verfahren nach Schritt d) einen weiteren Schritt umfaßt, in dem die Bindung der unbehandelten Tumor- und/oder Immunzellen an organspezifische Endothelzellen und/oder an organspezifische extrazelluläre Strukturen detektiert wird, in dem die Bindung der mit dem mindestens einen in Schritt d) identifizierten Aminopeptidasen-Inhibitor behandelten Tumor- und/oder Immunzellen an die organspezifischen Endothelzellen und/oder die organspezifischen extrazellulären Strukturen detektiert wird und in dem die detektierten Bindungen verglichen werden.

4. Verfahren zur Identifikation mindestens eines Inhibitors, der in Kombination mit mindestens einem Aminopeptidasen-Inhibitor eine Blockierung einer Polarisierung von invasiven menschlichen oder tierischen Tumor- und/oder Immunzellen bewirkt, folgende Schritte umfassend:
a) Detektieren von Oberflächenproteinkombinationen eines Proteinnetzwerkes, die sich auf der Oberfläche der unbehandelten Tumorund/oder Immunzellen befinden, wobei das Proteinnetzwerk bis zu 30 Oberflächenproteine aus einer Gruppe bestehend aus
| | | | | |
|---|---|---|---|---|
| 1. CD4 | 2.CD8 | 3. HLA-DR | 4. HLA-DQ | 5. CD3 |
| 6. CD26 | 7. CD38 | 8.CD45RA | 9. CD16 | 10. CD57 |
| 11. CD56 | 12. CD7 | 13. CD54 | 14. CD58 | 15. CD138 |
| 16. CD13 | 17. CD62L | 18. CD71 | 19. CD11 b | 20. CD36 |
| 21. CD29 | 22. CD49d | 23. CD18 | 24. CD49f | 25. CD19 |
| 26. CD2 | 27. CD20 | 28. CD10 | 29. CD44 | 30. CD80 |
umfaßt;
b) Behandeln der oder gleichartiger Tumor- und/oder Immunzellen mit mindestens einem potentiellen Inhibitor, der nicht gegen eine Aminopeptidase gerichtet ist;
c) Detektieren der Oberflächenproteinkombinationen des Proteinnetzwerkes, die sich auf der Oberfläche der behandelten Tumor- und/oder Immunzellen befinden; und
d) Vergleichen der in Schritt a) und in Schritt c) detektierten Oberflächenproteinkombinationen, wobei der mindestens eine Inhibitor bei einer Abweichung der in Schritt a) detektierten Oberflächenproteinkombinationen von den in Schritt c) detektierten Oberflächenproteinkombinationen durch mindestens eine Veränderung eines Oberflächenproteins zur Blockierung der Polarisierung der Tumorund/oder Immunzellen geeignet ist.

5. Verfahren nach Anspruch 4,
**dadurch gekennzeichnet,**
**daß** die oder die gleichartigen Tumor- und/oder Immunzellen in Schritt b) auch mit mindestens einem Aminopeptidasen-Inhibitor behandelt werden, wobei die Kombination von dem mindestens einen Inhibitor und dem mindestens einen Aminopeptidasen-Inhibitor bei einer Abweichung der in Schritt a) detektierten Oberflächenproteinkombinationen von den in Schritt c) detektierten Oberflächenproteinkombinationen durch mindestens eine Veränderung eines Oberflächenproteins CD13 die Blockierung der Polarisierung der Tumor- und/oder Immunzellen bewirkt.

6. Verfahren nach einem der Ansprüche 4 oder 5,
**dadurch gekennzeichnet,**
**daß** das Verfahren nach Schritt d) einen weiteren Schritt umfaßt, in dem der mindestens eine in Schritt d) identifizierte Inhibitor oder eine Kombination aus dem mindestens einen in Schritt d) identifizierten Inhibitor und mindestens einem Aminopeptidasen-Inhibitor zu mindestens einer polarisierenden Tumor- und/oder Immunzelle zugegeben wird und die weitere Entwicklung der mindestens einen polarisierenden Tumor- und/oder Immunzelle detektiert wird.

7. Verfahren nach einem der Ansprüche 4 bis 6,
**dadurch gekennzeichnet,**
**daß** das Verfahren nach Schritt d) einen weiteren Schritt umfaßt, in dem die Bindung der unbehandelten Tumor- und/oder Immunzellen an organspezifische Endothelzellen und/oder an organspezifische extrazelluläre Strukturen detektiert wird, in dem die Bindung der mit dem mindestens einen in Schritt d) identifizierten Inhibitor oder mit einer Kombination aus dem mindestens einen in Schritt d) identifizierten Inhibitor und mindestens einem Aminopeptidasen-Inhibitor behandelten Tumor- und/oder Immunzellen an die organspezifischen Endothelzellen und/oder die organspezifischen extrazellulären Strukturen detektiert wird und in dem die detektierten Bindungen verglichen werden.
